# EUROPEAN PATENT SPECIFICATION

(11) **EP 1 446 383 B1**
(45) Date of publication and mention of the grant of the patent: **17.09.2008**
(21) Application number: 02783080.1
(22) Date of filing: 11.11.2002
(51) Int. Cl.: C07D 223/28, A61K 31/55, A61P 25/24

(54) **MONOHYDROXYCARBAMAZEPINE FOR USE IN THE PREPARATION OF A MEDICAMENT FOR THE TREATMENT OF AFFECTIVE AND ATTENTION DISORDER AND NEUROPATHIC PAIN**
MONOHYROXYCARBAMEZEPIN ZUR VERWENDUNG BEI DER HERSTELLUNG EINES MEDIKAMENTS ZUR BEHANDLUNG VON AFFEKTIVER PSYCHOSE, AUFMERKSAMKEITS STÖRUNGEN UND NEUROPATHISCHEN SCHMERZEN
UTILISATION DE MONOHYDROXYCARBAMAZEPINE POUR PREPARER UN MEDICAMENT DESTINE AU TRAITEMENT DE TROUBLES AFFECTIFS ET DE L'ATTENTION ET DE DOULEURS NEUROPATHIQUES

(30) Priority: 12.11.2001 GB 0127177; 12.11.2001 GB 0127176; 12.11.2001 GB 0127178
(43) Date of publication of application: 18.08.2004
(73) Proprietor: Novartis AG, 4056 Basel (CH); Novartis Pharma GmbH, 1235 Vienna (AT)
(72) Inventor: SCHMUTZ, Markus, CH-4124 Schoenenbuch (CH)
(74) Representative: Harrison Goddard Foote
(86) International application number: PCT/EP2002/012578
(87) International publication number: WO 2003/042182

(56) References cited:
- GB-A- 1 310 120
- CZUCZWAR S J ET AL: "THE NEW GENERATION OF GABA ENHANCERS POTENTIAL IN THE TREATMENT OF EPILEPSY" CNS DRUGS, ADIS INTERNATIONAL, AUCKLAND, NZ, vol. 15, no. 5, 2001, pages 339-350, XP001095132 ISSN: 1172-7047

## Description

The present invention relates to a new use of a carbamazepine derivative.

More particularly, the present invention relates to the use of a compound of the formula 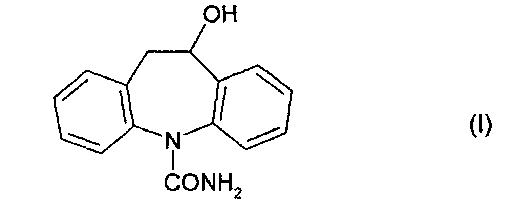 for the manufacture of a medicament for the treatment of a disorder, which disorder is selected from the group consisting of affective disorders and attention disorders.

Monohydroxycarbamazepine (10-hydroxy-10,11-dihydro-carbamazepine) of the formula I, the main metabolite of the antiepileptic oxcarbazepine (Trileptal ^{®}), is well-known from the literature [see for example Schuetz H. et al., Xenobiotica (GB), 16(8), 769-778 (1986)] and can be prepared synthetically starting from oxcarbazepine according to conventional methods. Monohydroxycarbamazepine has been first disclosed in GB 1310120. The compound is indicated to be suitable for the treatment of psychosomatic disturbances, epilepsy, trigeminal neuralgia and cerebral spasticity.

In accordance with the present invention, it has now surprisingly been found that a compound of the formula I is useful for the manufacture of a medicament for the treatment of affective disorders and attention disorders, including, e.g, depression and bipolar mood disorders.

The activity of a compound of the formula I in the treatment of affective disorders and attention disorders is evidenced, for example, by its ability to inhibit gamma- aminobutyric acid (GABA) turnover. This is due to feedback inhibition caused by the activating effect of a compound of the formula I on GABA transmission.

The role of GABA in bipolar and other mood disorders is unquestioned and topic of several reviews (e.g., Emrich et al., Effect of sodium valproate on mania. The GABA-hypothesis of affective disorders. Arch. Psychiatr. Nervenkr. 1980; 229:1-16; Petty. GABA and mood disorders: a brief review and hypothesis. J. Affect. Disord. 1995; 34:275-81). Drugs effective in (bipolar) mood disorders and also in anxiety and depression, e.g. lithium, valproate and diazepam, are known to inhibit the GABA turnover rate. This is due to feedback inhibition caused by the activating effect of these compounds on GABA transmission.

The activity of a compound of the formula I on GABA turnover is evidenced in the following experiment:

The determination of GABA turnover is based on the linear increase in GABA level observed after the maximal inhibition of gamma- aminobutyric acid transaminase (GABA-T). The values obtained with this approach for the rate of GABA synthesis are independent of the inhibitors used and within the catalytic capacity of the enzymes involved in the GABA shunt.

Under these conditions, a compound of the formula I dose-dependently inhibits the GABA turnover rate at doses of from 30 to about 300 mg/kg p.o..

The activity of a compound of the formula I in the treatment of affective disorders and attention disorders is also evidenced, for example, in tests suitable for detecting drugs having potential behavioural desinhibitory and/or sociotropic effects, which are thought to be relevant for recovery from social withdrawal, a cardinal feature of depression and related psychiatric conditions. For instance, drug effects on social withdrawal of intruder mice can be evaluated by using the basic method as described in Triangle, 1982, 21:95-105, and J. Clin. Psychiatry, 1994, 55:9 (suppl. B) 4-7.

Within the dose range of from 1 to about 100 mg/kg p.o., a compound of the formula I increases the social investigation in the treated mouse under such experimental conditions.

In view of its anxiolytic-/antidepressant- like stimulating effect on GABA transmission and sociotropic activity, a compound of the formula I is useful for the manufacture of a medicament treatment of affective disorders, including depression and bipolar disorders, e.g. manic-depressive psychoses, extreme psychotic states, e.g. mania, schizophrenia, and excessive mood swings where behavioural stabilization is desired. In addition, a compound of the formula I is indicated in ADHD (attention deficit hyperactivity disorders) and other attention disorders, e.g. autism, (and) in anxiety states, generalized anxiety and agoraphobia, as well as those behavioural states characterised by social withdrawal, e.g. negative symptoms.

For the above-mentioned indications, the appropriate dosage will of course vary depending upon, for example, the compound of the formula I employed, the host, the mode of administration and the nature and severity of the condition being treated. However, in general, satisfactory results in animals are indicated to be obtained at a daily dosage of from about 0.05 to about 150, preferably from about 0.1 to about 100, mg/kg of animal body weight. In larger mammals, for example humans, an indicated daily dosage is in the range of from about 0.5 to about 5000, preferably from about 1 to about 500, mg of a compound of the formula I, conveniently administered, for example, in divided doses up to four times a day or in sustained release form, for example once a day.

A compound of the formula I may be administered by any conventional route, in particular enterally, preferably orally, for example in the form of tablets or capsules, or parenterally, for example in the form of injectable solutions or suspensions.

A compound of the formula I can be administered in vivo either alone or in combination with other pharmaceutical agents, e.g. agents effective in the treatment of diseases and conditions in which the human VR1 activation plays a role or is implicated, including cyclooxygenase-2 (COX-2) inhibitors, such as specific COX-2 inhibitors (e.g. celecoxib, COX189 and rofecoxib) or in general nonsteroidal anti-inflammatory drugs (NSAIDs) (e.g. acetylsalicylic acid, propionic acid derivatives), tricyclic antidepressants (e.g. Anafranil^{®}, Asendin^{®}, Aventyl^{®}, Elavil^{®}, Endep^{®}, Norfranil^{®}, Norpramin^{®}, Pamelor^{®}, Sinequan^{®}, Surmontil^{®}, Tipramine^{®}, Tofranil^{®}, Vivactil^{®}, Tofranil-PM^{®}), anticonvulsants (e.g. gabapentin), GABA_{B} agonists (e.g. L-baclofen), opioids, vanilloid receptor antagonists and cannabinoid (CB) receptor agonists, e.g. CB₁, receptor agonists.

The pharmaceutical compositions for separate administration of the combination partners and for the administration in a fixed combination, i.e. a single galenical composition comprising at least two combination partners, according to the invention can be prepared in a manner known per se and are thus suitable for enteral, such as oral or rectal, and parenteral administration to mammals, including man, comprising a therapeutically effective amount of at least one pharmacologically active combination partner alone or in combination with one or more pharmaceutically acceptable carriers, especially suitable for enteral or parenteral application.

## Claims

1. The use of a compound of the formula: 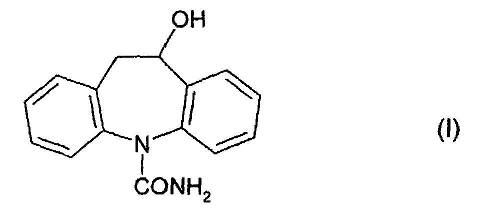 for the manufacture of a medicament for the treatment of a disorder, which disorder is selected from the group consisting of affective disorders and attention disorders.

2. The use according to claim 1, **characterised in that** the disorder is selected from the group consisting of affective disorders.

3. The use according to claim 2, **characterised in that** the disorder is depression.

4. The use according to claim 2, **characterised in that** the disorder is selected from the group consisting of bipolar disorders.

5. The use according to claim 2, **characterised in that** the disorder is selected from the group consisting of bipolar mood disorders.

6. The use according to claim 2, **characterised in that** the disorder is selected from the group consisting of manic-depressive psychoses.

7. The use according to claim 2, **characterised in that** the disorder is selected from the group consisting of extreme psychotic states.

8. The use according to claim 2, **characterised in that** the disorder is mania.

9. The use according to claim 2, **characterised in that** the disorder is schizophrenia.

10. The use according to claim 2, **characterised in that** the disorder is selected from the group consisting of excessive mood swings, where behavioural stabilization is desired.

11. The use according to claim 1, **characterised in that** the disorder is selected from the group consisting of attention disorders.

12. The use according to claim 11, **characterised in that** the disorder is selected from the group consisting of attention deficit hyperactivity disorders (ADHD).

13. The use according to claim 11, **characterised in that** the disorder is autism.

14. The use according to claim 1, **characterised in that** the disorder is selected from the group consisting of anxiety states.

15. The use according to claim 1, **characterised in that** the disorder is generalized anxiety.

16. The use according to claim 1, **characterised in that** the disorder is agoraphobia.

17. The use according to claim 1, **characterised in that** the disorder is selected from the group consisting of behavioural states **characterised by** social withdrawal.

18. The use according to claim 17, **characterised in that** the disorder is selected from the group consisting of negative symptoms.

19. The use according to claim 1, **characterised in that** the medicament is a combination comprising at least one other pharmacologically active compound in addition to a compound of the formula I.

20. The use according to claim 19, **characterised in that** the combination is a fixed combination.

21. A process for the manufacture of a medicament for the treatment of a disorder, which disorder is selected from the group consisting of affective disorders and attention disorders, which process is **characterised in that** a compound of the formula: 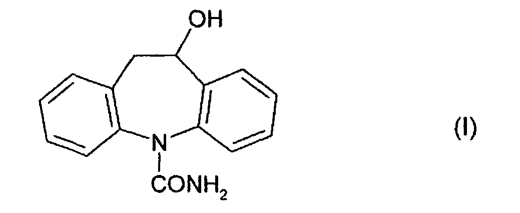 is used as pharmacologically active compound in association with at least one pharmaceutically acceptable carrier or diluent.

22. A process according to claim 21, **characterised in that** the disorder is selected from the group consisting of affective disorders.

23. A process according to claim 22, **characterised in that** the disorder is depression.

24. A process according to claim 22, **characterised in that** the disorder is selected from the group consisting of bipolar disorders.

25. A process according to claim 22, **characterised in that** the disorder is selected from the group consisting of bipolar mood disorders.

26. A process according to claim 22, **characterised in that** the disorder is selected from the group consisting of manic-depressive psychoses.

27. A process according to claim 22, **characterised in that** the disorder is selected from the group consisting of extreme psychotic states.

28. A process according to claim 22, **characterised in that** the disorder is mania.

29. A process according to claim 22, **characterised in that** the disorder is schizophrenia.

30. A process according to claim 22, **characterised in that** the disorder is selected from the group consisting of excessive mood swings, where behavioural stabilization is desired.

31. A process according to claim 21, **characterised in that** the disorder is selected from the group consisting of attention disorders.

32. A process according to claim 31, **characterised in that** the disorder is selected from the group consisting of attention deficit hyperactivity disorders (ADHD).

33. A process according to claim 31, **characterised in that** the disorder is autism.

34. A process according to claim 21, **characterised in that** the disorder is selected from the group consisting of anxiety states.

35. A process according to claim 21, **characterised in that** the disorder is generalized anxiety.

36. A process according to claim 21, **characterised in that** the disorder is agoraphobia.

37. A process according to claim 21, **characterised in that** the disorder is selected from the group consisting of behavioural states **characterised by** social withdrawal.

38. A process according to claim 37, **characterised in that** the disorder is selected from the group consisting of negative symptoms.

39. A process according to claim 21, **characterised in that** the medicament is a combination comprising at least one other pharmacologically active compound in addition to a compound of the formula 1.

40. A process according to claim 39, **characterised in that** the combination is a fixed combination.

## Patentansprüche

1. Verwendung einer Verbindung der Formel 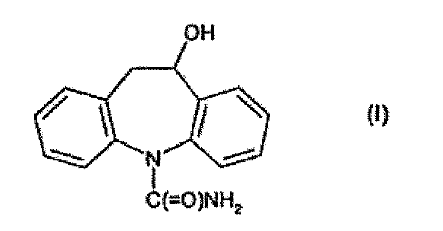 für die Herstellung eines Arzneimittels für die Behandlung einer Störung, wobei die Störung gewählt ist aus der Gruppe, die besteht aus Gemütsstörungen und Aufmerksamkeits-Störungen.

2. Verwendung nach Anspruch 1, **dadurch gekennzeichnet, dass** die Störung gewählt ist aus der Gruppe, die besteht aus Gemütsstörungen.

3. Verwendung nach Anspruch 2, **dadurch gekennzeichnet, dass** die Störung eine Depression ist.

4. Verwendung nach Anspruch 2, **dadurch gekennzeichnet, dass** die Störung gewählt ist aus der Gruppe, die besteht aus bipolaren Störungen.

5. Verwendung nach Anspruch 2, **dadurch gekennzeichnet, dass** die Störung gewählt ist aus der Gruppe, die besteht aus bipolaren Stimmungsstörungen.

6. Verwendung nach Anspruch 2, **dadurch gekennzeichnet, dass** die Störung gewählt ist aus der Gruppe, die besteht aus manisch depressiven Psychosen.

7. Verwendung nach Anspruch 2, **dadurch gekennzeichnet, dass** die Störung gewählt ist aus der Gruppe, die besteht aus extremen psychotischen Zuständen.

8. Verwendung nach Anspruch 2, **dadurch gekennzeichnet, dass** die Störung eine Manie ist.

9. Verwendung nach Anspruch 2, **dadurch gekennzeichnet, dass** die Störung Schizophrenie ist.

10. Verwendung nach Anspruch 2, **dadurch gekennzeichnet, dass** die Störung gewählt ist aus der Gruppe, die besteht aus übermäßigen Stimmungsschwankungen, bei denen eine Stabilisierung des Verhaltens gewünscht ist.

11. Verwendung nach Anspruch 1, **dadurch gekennzeichnet, dass** die Störung gewählt ist aus der Gruppe, die besteht aus Aufrnerksamkeitsstörungen.

12. Verwendung nach Anspruch 11, **dadurch gekennzeichnet, dass** die Störung gewählt ist aus der Gruppe, die besteht aus Aufmerksamkeitsdefizit-Hyperaktivitäts-Störungen (attention deficit hyperactivity disorders (ADHD)).

13. Verwendung nach Anspruch 11, **dadurch gekennzeichnet, dass** die Störung Autismus ist.

14. Verwendung nach Anspruch 1, **dadurch gekennzeichnet, dass** die Störung gewählt ist aus der Gruppe, die besteht aus Angst-Zuständen.

15. Verwendung nach Anspruch 1, **dadurch gekennzeichnet, dass** die Störung allgemeine Angst ist.

16. Verwendung nach Anspruch 1, **dadurch gekennzeichnet, dass** die Störung Agoraphobie ist.

17. Verwendung nach Anspruch 1, **dadurch gekennzeichnet, dass** die Störung gewählt ist aus der Gruppe, die besteht aus Verhaltenszuständen, die durch Rückzug aus dem sozialen Umfeld **gekennzeichnet** sind.

18. Verwendung nach Anspruch 17, **dadurch gekennzeichnet, dass** die Störung gewählt ist aus der Gruppe, die besteht aus negativen Symptomen.

19. Verwendung nach Anspruch 1, **dadurch gekennzeichnet, dass** das Arzneimittel eine Kombination ist, die wenigstens eine andere pharmakologisch aktive Verbindung zusätzlich zu einer Verbindung der Formel (I) umfasst.

20. Verwendung nach Anspruch 19, **dadurch gekennzeichnet, dass** die Kombination eine feste Kombination ist.

21. Verfahren zur Herstellung eines Medikaments zur Behandlung einer Störung, wobei die Störung gewählt ist aus der Gruppe, die besteht aus Gemütsstörungen und Aufmerksamkeitsstörungen, wobei das Verfahren **dadurch gekennzeichnet ist, dass** eine Verbindung der Formel 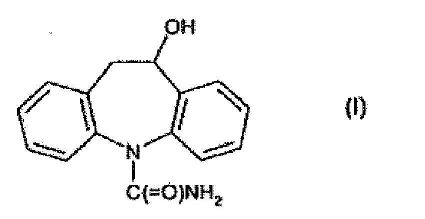 als pharmakologisch aktive Verbindung zusammen mit wenigstens einem pharmazeutisch annehmbaren Träger oder Verdünnungsmittel verwendet wird.

22. Verfahren nach Anspruch 21, **dadurch gekennzeichnet, dass** die Störung gewählt ist aus der Gruppe, die besteht aus Gemütsstörungen.

23. Verfahren nach Anspruch 22, **dadurch gekennzeichnet, dass** die Störung eine Depression ist.

24. Verfahren nach Anspruch 22, **dadurch gekennzeichnet, dass** die Störung gewählt ist aus der Gruppe, die besteht aus bipolaren Störungen.

25. Verfahren nach Anspruch 22, **dadurch gekennzeichnet, dass** die Störung gewählt ist aus der Gruppe, die besteht aus bipolaren Stimmungsstörungen.

26. Verfahren nach Anspruch 22, **dadurch gekennzeichnet, dass** die Störung gewählt ist aus der Gruppe, die besteht aus manisch-depressiven Psychosen.

27. Verfahren nach Anspruch 22, **dadurch gekennzeichnet, dass** die Störung gewählt ist aus der Gruppe, die besteht aus extremen psychotischen Zuständen.

28. Verfahren nach Anspruch 22, **dadurch gekennzeichnet, dass** die Störung eine Manie ist.

29. Verfahren nach Anspruch 22, **dadurch gekennzeichnet, dass** die Störung Schizophrenie ist.

30. Verfahren nach Anspruch 22, **dadurch gekennzeichnet, dass** die Störung gewählt ist aus der Gruppe, die besteht aus übermäßigen Stimmungsschwankungen, bei denen eine Verhaltensstabilisierung gewünscht ist.

31. Verfahren nach Anspruch 21, **dadurch gekennzeichnet, dass** die Störung gewählt ist aus der Gruppe, die besteht aus Aufmerksamkeits-Störungen.

32. Verfahren nach Anspruch 31, **dadurch gekennzeichnet, dass** die Störung gewählt ist aus der Gruppe, die besteht aus Aufmerksamkeits-Defizit-Hyperaktivitäts-Störungen (attention deficit hyperactivity disorders (ADHD)).

33. Verfahren nach Anspruch 31, **dadurch gekennzeichnet, dass** die Störung Autismus ist.

34. Verfahren nach Anspruch 21, **dadurch gekennzeichnet, dass** die Störung gewählt ist aus der Gruppe, die besteht aus Angst-Zuständen.

35. Verfahren nach Anspruch 21, **dadurch gekennzeichnet, dass** die Störung allgemeine Angst ist.

36. Verfahren nach Anspruch 21, **dadurch gekennzeichnet, dass** die Störung Agoraphobie ist.

37. Verfahren nach Anspruch 21, **dadurch gekennzeichnet, dass** die Störung gewählt ist aus der Gruppe, die besteht aus Verhaltenszuständen, die durch Rückzug aus dem sozialen Umfeld charakterisiert sind.

38. Verfahren nach Anspruch 37, **dadurch gekennzeichnet, dass** die Störung gewählt ist aus der Gruppe, die besteht aus negativen Symptomen.

39. Verfahren nach Anspruch 21, **dadurch gekennzeichnet, dass** das Arzneimittel eine Kombination ist, die wenigstens eine weitere pharmakologisch aktive Verbindung zusätzlich zu einer Verbindung der Formel (I) umfasst.

40. Verfahren nach Anspruch 39, **dadurch gekennzeichnet, dass** die Kombination eine feste Kombination ist.

## Revendications

1. L'utilisation du composé de formule: 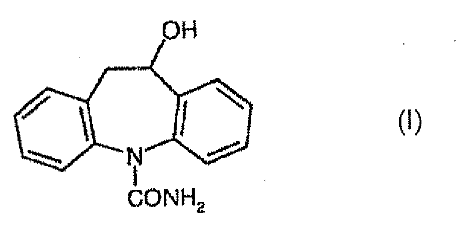 pour la fabrication d'un médicament pour le traitement d'un trouble, lequel trouble est choisi dans le groupe consistant en des troubles affectifs et des troubles de l'attention.

2. L'utilisation selon la revendication 1, **caractérisée en ce que** le trouble est choisi dans le groupe consistant en des troubles affectifs.

3. L'utilisation selon la revendication 2, **caractérisée en ce que** le trouble est une dépression.

4. L'utilisation selon la revendication 2, **caractérisée en ce que** le trouble est choisi dans le groupe consistant en des troubles bipolaires.

5. L'utilisation selon la revendication 2, **caractérisée en ce que** le trouble est choisi dans le groupe consistant en des troubles bipolaires de l'humeur.

6. L'utilisation selon la revendication 2, **caractérisée en ce que** le trouble est choisi dans le groupe consistant en des psychoses maniaco-dépressives.

7. L'utilisation selon la revendication 2, **caractérisée en ce que** le trouble est choisi dans le groupe consistant en des états psychotiques extrêmes.

8. L'utilisation selon la revendication 2, **caractérisée en ce que** le trouble est une manie.

9. L'utilisation selon la revendication 2, **caractérisée en ce que** le trouble est la schizophrénie.

10. L'utilisation selon la revendication 2, **caractérisée en ce que** le trouble est choisi dans le groupe consistant en des oscillations excessives de l'humeur, lorsque la stabilisation comportementale est souhaitée.

11. L'utilisation selon la revendication 1, **caractérisée en ce que** le trouble est choisi dans le groupe consistant en des troubles de l'attention.

12. L'utilisation selon la revendication 11, **caractérisée en ce que** le trouble est choisi dans le groupe consistant en des troubles d'hyperactivité avec déficit d'attention (ADHD).

13. L'utilisation selon la revendication 11, **caractérisée en ce que** le trouble est l'autisme.

14. L'utilisation selon la revendication 1, **caractérisée en ce que** le trouble est choisi dans le groupe consistant en des états d'anxiété.

15. L'utilisation selon la revendication 1, **caractérisée en ce que** le trouble est de l'anxiété généralisée.

16. L'utilisation selon la revendication 1, **caractérisée en ce que** le trouble est l'agoraphobie.

17. L'utilisation selon la revendication 1, **caractérisée en ce que** le trouble est choisi dans le groupe consistant en des états comportementaux **caractérisés par** un retrait social.

18. L'utilisation selon la revendication 17, **caractérisée en ce que** le trouble est choisi dans le groupe consistant en des symptômes négatifs.

19. L'utilisation selon la revendication 1, **caractérisée en ce que** le médicament est une combinaison comprenant au moins un autre composé pharmacologiquement actif en addition à un composé de formule I.

20. L'utilisation selon la revendication 19, **caractérisée en ce que** la combinaison est une combinaison fixée.

21. Un procédé pour la fabrication d'un médicament pour le traitement d'un trouble, lequel trouble est choisi dans le groupe consistant en des troubles affectifs et des troubles de l'attention, lequel procédé est **caractérisé en ce qu'**un composé de formule : 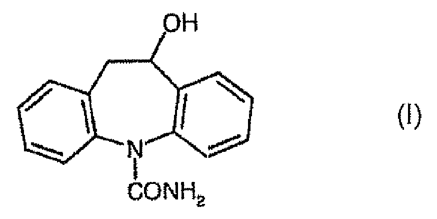 est utilisé comme composé pharmacologiquement actif en association avec au moins un porteur ou un diluant pharmaceutiquement acceptable.

22. Un procédé selon la revendication 21, **caractérisé en ce que** le trouble est choisi dans le groupe consistant en des troubles affectifs.

23. Un procédé selon la revendication 22, **caractérisé en ce que** le trouble est une dépression.

24. Un procédé selon la revendication 22, **caractérisé en ce que** le trouble est choisi dans le groupe consistant en des troubles bipolaires.

25. Un procédé selon la revendication 22, **caractérisé en ce que** le trouble est choisi dans le groupe consistant en des troubles bipolaires de l'humeur.

26. Un procédé selon la revendication 22, **caractérisé en ce que** le trouble est choisi dans le groupe consistant en des psychoses maniaco-dépressives.

27. Un procédé selon la revendication 22, **caractérisé en ce que** le trouble est choisi dans le groupe consistant en des états psychotiques extrêmes.

28. Un procédé selon la revendication 22, **caractérisé en ce que** le trouble est une manie.

29. Un procédé selon la revendication 22, **caractérisé en ce que** le trouble est la schizophrénie.

30. Un procédé selon la revendication 22, **caractérisé en ce que** le trouble est choisi dans le groupe consistant en des oscillations excessives de l'humeur, lorsque la stabilisation comportementale est souhaitée.

31. Un procédé selon la revendication 21, **caractérisé en ce que** le trouble est choisi dans le groupe consistant en des troubles de l'attention.

32. Un procédé selon la revendication 31, **caractérisé en ce que** le trouble est choisi dans le groupe consistant en des troubles d'hyperactivité avec déficit d'attention (ADHD).

33. Un procédé selon la revendication 31, **caractérisé en ce que** le trouble est l'autisme.

34. Un procédé selon la revendication 21, **caractérisé en ce que** le trouble est choisi dans le groupe consistant en des états d'anxiété.

35. Un procédé selon la revendication 21, **caractérisé en ce que** le trouble est de l'anxiété généralisée.

36. Un procédé selon la revendication 21, **caractérisé en ce que** le trouble est l'agoraphobie.

37. Un procédé selon la revendication 21, **caractérisé en ce que** le trouble est choisi dans le groupe consistant en des états comportementaux **caractérisés par** un retrait social.

38. Un procédé selon la revendication 37, **caractérisé en ce que** le trouble est choisi dans le groupe consistant en des symptômes négatifs.

39. Un procédé selon la revendication 21, **caractérisé en ce que** le médicament est une combinaison comportant au moins un autre composé pharmacologiquement actif en addition à un composé de formule 1.

40. Un procédé selon la revendication 39, **caractérisé en ce que** la combinaison est une combinaison fixée.
